## Europäisches Patentamt

## European Patent Office

## Office européen des brevets

(19)

(11) Veröffentlichungsnummer: **0 135 604**
**B1**

(12)

# EUROPÄISCHE PATENTSCHRIFT

(45) Veröffentlichungstag der Patentschrift:
26.11.86

(51) Int. Cl.⁴: **C 07 C 103/19, C 12 P 29/00**

(21) Anmeldenummer: **83109643.3**

(22) Anmeldetag: **27.09.83**

(54) **Verfahren zur Herstellung eines Oxytetracyclin-Calciumsilikat-Komplexsalzes aus Fermentbrühe.**

(43) Veröffentlichungstag der Anmeldung:
**03.04.85 Patentblatt 85/14**

(45) Bekanntmachung des Hinweises auf die Patenterteilung:
**26.11.86 Patentblatt 86/48**

(84) Benannte Vertragsstaaten:
**AT BE CH DE FR GB IT LI NL SE**

(56) Entgegenhaltungen:
GB-A-718 020
GB-A-1 132 124

CHEMICAL ABSTRACTS, Band 87, Nr. 22, 28.
November 1977, Seite 314, Nr. 172905m, Columbus,
Ohio, USA

(73) Patentinhaber: **BIOGAL GYOGYSZERGYAR, Pallagi
u. 13., H-4042 Debrecen (HU)**

(72) Erfinder: **Bálint, János, Dr., Péchy u. 5, Debrecen
(HU)**
Erfinder: **Cséke, László, Hajo u.4, Debrecen (HU)**
Erfinder: **Fábián, Ferenc, Holló I.u.4, Debrecen (HU)**
Erfinder: **Jun, Lajos, Szegfü u.6, Debrecen (HU)**
Erfinder: **Szarvas, Miklós, Dr., Péterfia u. 40,
Debrecen (HU)**

(74) Vertreter: **Kraus, Walter, Dr., Patentanwälte
Kraus, Weisert & Partner Thomas- Wimmer-
Ring 15, D-8000 München 22 (DE)**

## Beschreibung

Die Erfindung betrifft ein einfaches, industrielles, auch in Eisenapparaten durchführbares Verfahran zur Herstellung eines Oxytetracyclin-Calciumsilikat-Komplexsalzes aus Fermentbrühe.

Das Oxytetracyclin (OTC) ist ein die Proteinsynthese und den Replikationsmechanismus der Mikroorganismen hemmendes Antiobiotikun mit breitem Wirkungsspektrum, das man zur Heilung von zahlreichan durch pathogene Mikroorganismen verursachten Krankheiten sowie wegen seiner die Gawichtszunahme steigernden und Krankheiten verhütenden Wirkung in der Landwirtschaft als Futtermittelzusatz verwendet. In industriellem Maßstabe wird OTC durch aerobe Farmentierung von Streptomyces rimosus in submerser Kultur, amschließendar Gewinnung des Antibiotikums aus der rohen Fermentbrühe und abschließender Reinigung hergestellt.

Zur Isolierung und Reinigung des OTC sind Zahlreiche Verfahren bekannt. Zum Beispiel kann OTC aus alkalischer Lösung mit Butylalkohol extrahiert werden (US-PS Nr. 2 516 080). Es kann ferner an Ionenaustauscherharz gebunden (US-PS Nr. 2 658 078), mit Barium-, Calcium- oder Magnesiumionen ausgefällt (J.Am.Chem.Soc. 73 /1951/ 4211: britische Patentschrift Nr. 718 020) sowie mit quaternären Ammoniumsalzen (US-PS Nr. 2 873 276) oder mit Arlazosulfonsäuren (US-PS Nr. 2 649 480) niedergeschlagen werden. Die aufgeführten Verfahren haben verschiedene Nachteile, zum Beispiel erfordern sie die Verwendung teurer Vorrichtungen wie Extraktoren Ionenaustauschersäulen oder teurer Hilfsstoffe (Ionenaustauscher, quaternäre Ammoniumsalze, Arylazosulfonsäuren)oder stark giftiger Verbindungen (Bariumsalze).

Auf den ersten Blick scheint das Verfahren gemäß der belgischen Patentschrift Nr. 632 331 wesentlicn einfacher und leichter ausführbar: das OTC wird in Gegenwart von Calciunionen mit Carbonat- oder Hydrogencarbonationen ausgefällt. Das Verfahren erwies sich jedoch in eigenen Versuchen als schwer reproduzierbar; die Ausbeute ist in der genannten Publikation nicht angegeben.

Ein ähnliches Verfahren (ungarische Patentschrift Nr. 173 703) ernöglicht eine maximale Ausbeute von 55% Gemäß der ungarischen Patentschrift Nr. 172 330 wird das OTC mit 85% iger Ausbeute auf das Mycel aufgefällt. Dadurch ist der Gehalt des Produktes an reinem Wirkstoff natürlich sehr niedrig (etwa 13%). Das ist für die Herstellung und die weitere Reinigung gleichermaßen unangenehm, weil mit sehr großen Volumina gearbeitet werden muß.

Die industrielle Herstellung, Isolierung und Reinigung des OTC wird durch den Eisengehalt der Fermentbrühe stark erschwert. Eisen gelangt mit den Bestandteilen des Nährmediums und von der Wand der Eisenfermentoren in die Fermentbrühe und katalysiert als oxydierendes Agens die Zersetzung des OTC, d.h. verringert die Menge des bereits synthetisierten Wirkstoffes (ungarische Patentschrift Nr. 143 609) und verschlechtert die Ausbeute der Reinigungsschritte (britische Patentschrift Nr. 718 020). Auch werden Farbe und Stabilität des Reinproduktes durch den Eisengehalt-ungünstig beeinflußt: es ist nämlich bekannt, daß die zwei- und dreiwertigen Metalle mit OTC stabile innere Komplexe bilden (J. Am.Chem. Soc. 73 /1951/, 4211), die sich nicht leicht zersetzen lassen. Eine Möglichkeit, den Eisengehalt der Fermentbrühe auf einem niedrigen Niveau zu halten, besteht in der Verwendung von Geräten aus Edelstahl. Diese Apparate sind jedoch zu teuer, so daß großenteils noch überall mit eisernen Gefäßen gearbeitet wird.

Die im Eisenfermentor hergestellte, OTC enthaltende Fermentbrühe ist stark eisenhaltig (etwa 30-60 gamma/ml, es können auch höhere Werte vorkommen).

Man ist auf verschiedene Weise bestrebt, die schädliche Wirkung des Eisens dadurch zu mindern, daß man es in lösliche, jedoch nicht dissoziierende Komplexe überführt: der bekannteste Komplexbildner für diesen Zweck ist Äthylendiaminotetraessigsäure (EDTA). Eine andere Möglichkeit besteht darin, das Eisen zum Beispiel mit Ascorbinsaure, Natriumformaldehydsulfoxylat oder Natriumdithionit zum zweiwertigen Eisen zu reduzieren,weil das zweiwertige Eisen mit OTC viel weniger stabile Komplexe bildet und die Oxydation des Wirkstoffes nicht katalysiert. Schließlich kann das Eisen auch ausgefällt werden, zum Beispiel mit Kalium-(hexacyanoferrat(II)) als unlösliches Eisen(II,III)(hexacyanoferrat(II)) (britische Patentschrift Nr. 718 020). Diese Verfahren haben dem Nachteil teure Reagentien zu erfordern (EDTA, Ascorbinsäure), die auch in großen Mengen angewandt die schädliche Wirkung des Eisens nicht völlig beseitigen können. Das zum Ausfällen des Eisens am geeignetste Reagens, das Kalium-(hexacyanoferrat(II)), hat sich in der Praxis nicht durchsetzen können, weil immer die Möglichkeit der Bildung von Cyanwasserstoff besteht.

Ziel der Erfindung war die Ausarbeitung eines Verfahrens, mit dem mittels einfacher Technologie und einfacher Vorrichtungen das OTC wirtschaftlicher, in größerer Ausbeute als bisher und gewünschtenfalls in einem höheren Reinheitsgrad gewonnen werden kann.

Die Erfindung beruht auf der Erkenntnis, daß OTC in Gegenwart von Calciumionen mit wasserlöslichen Silikaten ein unlösliches, stabiles, leicht filtrierbares Komplexsalz bildet.

Gegenstand der Erfindung ist demnach ein Verfahren zur Herstellung eines Oxytetracyclin-Komplexsalzes aus der Fermentbrühe. Erfindungsgemäß geht man so vor, daß man die gegebenenfalls mit einer oberflächenaktiven Substanz vorbehandelte Fermentbrühe

a) nach einer gegebenenfalls durchgeführten Vorbehandlung mit einem wasserlöslichen Silikat auf pH 0,5-3,0 ansäuert, danach - gegebenenfalls unter Verwendung einer Filtrierhilfe - vom Mycel abfiltriert, den pH-Wert des Filtrates mit Lauge auf 4-5 einstellt und nach Zugabe eines Calciumsalzes oder in Gegenwart von Calciumionen ein wasserlösliches Silikat zusetzt, gegebenenfalls ein wasserlösliches Carbonat oder Hydrogencarbonat zugibt und nach längerem Rühren das gebildete Oxytetracyclin-Calciumsilikat-Komplexsalz gegebenenfalls in Gegenwart eines Filtrierhilfsstoffes abfiltriert, oder

b) mit einem Calciumsalz und einem wasserlöslichen Silikat vermischt, erforderlichenfalls den pH-Wert mit einer wasserlöslichen Lauge auf 8-11 einstellt, gegebenenfalls dem Gemisch ein wasserlösliches Carbonat oder Hydrogencarbonat zusetzt und nach längerem Rühren das auf das Mycel aufgefällte Oxytetracyclin-Calciumsilikat-Komplexsalz gegebenenfalls in Gegenwart eines Filtrierhilfsstoffes abfiltriert, und das nach a) oder b) erhaltene Oxytetracyclin-Calciumsilikat-Komplexsalz bei 20-120 °C trocknet.

Gemäß der Variante a) wird das Mycel nach dem Ansäuern abfiltriert, und der Calciumsilikatkomplex wird im klaren Filtrat gebildet. Gemäß der Verfahrensvariante b) wird der Komplex direkt auf das Mycel aufgefällt. Die Variante b) besteht aus weniger Schritten, jedoch ist ihr Produkt nur für Futterzwecke geeignet. Gemäß der Variante a) kann gewünschtenfalls ein eisenfreies, sehr reines Produkt erhalten werden, das für die Pharmazie weiterverarbeitet werden kann.

Das Verfahren wird an Hand des Fließschemas näher erläutert.

Bevor die Aufarbeitung beginnt, kann die Fermentbrühe gegebenenfalls mit einer oberflächenaktiven Substanz vorbehandelt werden. Geeignet ist zum Beispiel das Sterogenol (Cetylpyridiniumbromid). Die oberflächenaktive Substanz zerstört im wesentlichen die Zellwände des Mikroorganismus (Streptomyces rimosus) und setzt dadurch auch das intracellular gebundene OTC frei. Dieser Plasmolyse genannte Prozeß verläuft bei Zusatz von 0,001-0,03 % (bezogen auf das Gewicht der Fermentbrühe), bei pH 5,3-5,6 und einer Temperatur von etwa 40 °C bis zur völligen Auflösung der Zellen. Die mit der genannten Menge Sterogenol behandelte Fermentbrühe ist unter dem Mikroskop zellenfrei.

Die Plasmolyse ist nicht in jedem Fall erforderlich. Sie wird im allgemeinen nur dann vorgenommen, wenn die Fermentbrühe sehr dick ist und sich schlecht rühren läßt, was besonders dann der Fall ist, wenn vor der Fermentierung zwecks Verhütung der Schaumbildung Fette oder Öle zugesetzt wurden. Im Falle solcher Fermentbrühen kann durch die Behandlung mit oberflächenaktiver Substanz die Ausbeute am OTC sehr erhöht werden, weil auch die in den Zellen eingeschlossene Menge, die anderenfalls verloren ginge, mit erfaßt wird.

Gemäß der Verfahrensvariante a) wird die Fermentbrühe nun gegebenenfalls mit einem wasserlöslichen Silikat vorbehandelt. Dieser Schritt wird dann vorgenommen, wenn ein eisenfreies Produkt erhalten werden soll. Auf ihr Gewicht bezogen werden der Fermentbrühe 0,1-3,0 %, vorzugsweise 1,5-2,0 % eines wasserlöslichen Silikates, zweckmäßig Natronwasserglas, zugesetzt. Durch die Silikationen werden sowohl Eisen(II) wie auch Eisen(III) in Form ihrer Silikate ausgefällt, und diese sind in schwachen Basen und in schwachen Säuren unlöslich. Die ausgefällten Eisensilikate werden später beim Filtrierprozeß zusammen mit dem Mycel abgetrennt.

Der folgende Schritt besteht im Ansäuern der Fermentbrühe auf pH 0,5-3,0, vorzugsweise 1,0-2,0. Dazu können organische und anorganische Säuren gleichermaßen verwendet werden. Die Verwendung von Phosphor- oder Essigsäure ist weniger ratsam, weil diese Säuren die Epimerisierung des OTC zu dem biologisch wirkungslosen 4-Epimer katalysieren. Salzsäure greift den Wirkstoff OTC zu sehr an. Am besten bewährt haben sich Oxalsäure und Schwefelsäure. Oxalsäure allein zu verwende ist aus Preisgründen ungünstig. Man verwendet zweckmäßig ein Gemisch beider, das zu etwa 90 % aus Schwefelsäure und 10 % aus Oxalsäure besteht. Ein Gemisch, das 11 Gew.-% Schwefelsäure und 1,5 Gew.-% Oxalsäure enthält, wird in einer Menge von auf 1 kg Fermentbrühe gerechnet 30-120 ml, vorzugsweisa 45-70 ml eingesetzt. Obwohl während des Ansäuerns eine gewisse Zersetzung des OTC vor sich geht (J.Am.Chem.Soc. 75, 5455 /1953/), lassen sich doch die Verluste durch geeignete Wahl der Bedingungen unter 6 % halten.

Nun wird das Mycel (falls Eisensilikat gefällt wurde, auch dieses) durch Filtrieren von der sauren Fermentbrühe abgetrennt. Dabei kann ein Filtrierhilfsstoff, zum Beispiel Perlit, verwendet werden. Das auf dem Filter befindliche Mycel wird mit Säure der genannten Zusammensetzung einmal gewaschen, die Waschflüssigkeit wird mit dem Filtrat vereinigt. Auf diese Weise bleiben im Mycel maximal 8 % des Gesamtwirkstoffes zurück. Durch weiteres Auswaschen konnte auch diese Menge noch erfaßt werden, jedoch fallen dabei zu große Flüssigkeitsvolumina an, deren weitere Verarbeitung problematisch wäre.

Aus dem sauren Filtrat wird das OTC mit einem Calciumsalz und einem wasserlöslichen Silikat, vorzugsweise Wasserglas, ausgefällt. Falls das Eisen nicht im ersten Schritt gefallt und dann entfernt wurde, fällt es jetzt zusammen mit dem Komplexsalz aus und bleibt in diesem. Geeignet ist ein pH-Wert, bei dem Eisen(II) und Eisen(III) bereits als Silikate ausfallen, das OTC jedoch noch nicht geschädigt wird. Optimal ist ein pH-Wert von 4-5. Dieser wird mit einem basischen Stoff, zweckmäßig mit Natriumhydroxyd eingestellt.

Die Geschwindigkeit, mit der die Fällungsreagenzien zugesetzt werden, beeinflußt die Teilchengröße des Niederschlages. Im allgemeinen ist eine mittlere Teilchengröße anzustreben, da aus zu kleinen Teilchen der Wirkstoff zum Teil wieder in Lösung geht, im Falle zu großer Teilchen die Ausfällung nicht vollständig ist.

Als Calciumsalz wird zweckmäßig Calciumchlorid eingesetzt. Als wasserlösliches Silikat kommt praktisch nur das handelsübliche Wasserglas (Natronwasserglas = Natriumsilikat) in Frage. Dieses wird zweckmäßig mit Wasser auf das Vierfache seines Volumens verdünnt. Von dieser Verdünnung werden auf 1 kg Fermentbrühe bezogen 1-20 ml, vorzugsweise 4-9 ml benötigt.

Um die Fällung vollständig zu machen, wird der pH-Wert auf 8-11 eingestellt. Dazu wird zweckmäßig 2 n Natronlauge verwendet. Dann wird das Gemisch 30 Mimuten bis 5 Stunden lang gerührt. Die Fällung verläuft schneller, wenn man noch ein Carbonat oder Hydrogencarbonat zusetzt. Es handelt sich wahrscheinlich um einen Aussalzeffekt. Am geeignetsten ist Natriumhydrogencarbonat, das zweckmäßig in Form eimer 10 %igen Lösung verwendet wird. Auf 1 kg Fermentbrühe rechnet man 0,5-10 g, vorzugsweise 1-3 g Hydrogencarbonat.

Dann wird der ausgefallene Komplex so schnell wie möglich von der Mutterlauge abgetrennt, damit sich der Wirkstoff nicht wieder auflöst. Den richtigen Zeitpunkt bestimmt man durch Messen des Titers der Mutterlauge. Hat der Titer einen minimalen Wert erreicht, so wird filtriert. Gegebenenfalls kann ein Filtrierhilfsstoff verwendet werden. Unter diesen Bedingungen bleiben in der Mutterlauge maximal 0,04-0,06 mg/ml OTC zurück, d.h. der Verlust durch in der Mutterlauge verbliebenen OTC liegt unter 1 %.

Das filterfeuchte OTC-Komplexsalz wird dann unter atmosphärischem Druck oder im Vakuum getrocknet. Die Trocknungstemperatur liegt zwischen 20 und 120 °C, vorzugsweise bei 100-110 °C. Durch dauerndes Rühren, Zerteilen und Wenden des Materials wird die Trocknungszeit so kurz wie möglich gehalten, da die in dem Komplexsalz enthaltenen alkalischen Stoffe (Na-, Ca-Verbindungen) den Wirkstoff angreifen können.

Gemäß der Verfahrensvariante b) wird die Fermentbrühe unmittelbar mit den Fällungsreagentien, d.h. dem Calciumsalz und dem wasserlöslichen Silikat, versetzt. Das wasserlösliche Silikat (Natronwasserglas) wird in diesem Falle im Verhältnis 1:1 mit Wasser verdünnt und auf 1 kg Fermentbrühe gerechnet in einer Menge von 5-100 ml, vorzugsweise 8-12 ml eingesetzt.

Wasserglas ist stark basisch. Sollte sich trotzdem der zum Fällen optimale pH-Wert von 8-11 nicht einstellen, so wird durch Zugabe einer wasserlöslichen Base alkalisch gestellt. Zur Beschleunigung des Fällungsvorganges kann ein Carbonat oder Hydrogencarbonat zugegeben werden. Menge und Form des Zusatzes stimmen mit dem unter a) Gesagten überein. Das direkt auf das Mycel aufgefällte Komplexsalz wird auf die unter a) beschriebene Weise abgetrennt und getrocknet.

Gemäß dem erfindungsgemäßen Verfahren kann auch in den weniger teuren Eisenfermemtoren ein OTC-Calciumsilikat-Komplexsalz hergestellt werden, dessen gesamter, mit verdünnten Säuren mobilisierbarer Eisengehalt 2-200 gamma/g beträgt. Dieses Produkt ist zur Herstellung von OTC-Hydrochlorid und OTC-Dihydrat geeignet. Ein weiterer Vorteil des erfindungsgemäßen Verfahrens besteht darin, daß die Ausbeute größer ist als bei den bekannten Verfahren: etwa 85 % des in der Fermentbrühe enthaltenen OTC können gewonnen werden. Das erhaltene Komplexsalz enthält nach der Variante a) etwa 50 % Wirkstoff, nach der Variante b) etwa 18-20 % Wirkstoff. Das gemäß b) erhaltene Produkt kann so, wie es ist, für Fütterungszwecke verwendet werden.

Die Erfindung wird im folgenden an Hand von Ausführungsbeispielen näher erläutert.

**Beispiel 1**

Zu 2132 g OTC-Fermentbrühe (enthaltend insgesamt 15,56 g OTC) werden unter Rühren bei Raumtemperatur 40 ml im Verhältnis 1:1 verdünntes Natronwasserglas gegeben (Natronwasserglas gemäß ungarischem Standard Nr. MSZ 929, 40-45 °B, und Wasser im Verhältnis 1:1). Danach setzt man 150 ml 10 %ige Calciumchloridlösung zu und stellt den pH-Wert mit im Verhältnis 1:1 verdünntem Natronwasserglas auf 9 ein, wozu etwa 200 ml erforderlich sind. Das Gemisch wird 2 Stunden lang gerührt, danach wird abfiltriert. 157,8 g trockenes Produkt werden erhalten, dieses enthält 14,517 g (9,2 %) reinen Wirkstoff, was auf die Ausgangsmenge bezogen einer Ausbeute von 93 % entspricht.

**Beispiel 2**

Zu 2076 g OTC-Fermentbrühe (enthaltend 16,320 g OTC) werden bei Raumtemperatur 45 ml im Verhältnis 1:1 verdünntes Natronwasserglas und dann 86 ml 10 %ige CaCl₂-Lösung gegeben. Anschließend wird der pH-Wert mit 2 n Natronlauge auf 9,2 eingestellt und das Gemisch mit 75 ml-l0 %iger Natriumhydrogencarbonatlösung versetzt. Das Gemisch wird 2 Stunden lang gerührt und nach Zugabe von 11 g Perlit filtriert. Das trockeme Produkt wiegt 83,0 g und enthält 15,520 9 (18,7 %) OTC, was einer Ausbeute von 95 % entspricht.

**Beispiel 3**

Zu 2042 g OTC-Fermentbrühe (enthaltend insgesamt 19,68 g OTC) werden unter Rühren bei Raumtemperatur 50 ml im Verhältnis 1:1 verdünntes Natronwasserglas gegeben. Dann wird der pH-Wert mit 25 %iger Schwefelsäure auf 1 eingestellt, wozu etwa 200 ml erforderlich sind. Das Gemisch wird 30 Minuten lang gerührt, dann mit 6 g Perlit versetzt, filtriert, und das Mycel auf dem Filter mit 800 ml Wasser gewaschen. Filtrat und Waschwasser werden vereinigt. Die erhaltenen 2700 ml Flüssigkeit enthalten 17,52 g OTC. Unter Rühren wird der pH-Wert mit 2n Natronlauge auf 4,5 eimgestellt. Dann werden die Fällungsreagentien: 160 ml 10 %ige Calciumchloridlösung und im Verhältnis 1:3 verdünntes Natronwasserglas zugesetzt. Von letzterem wird so viel eingesetzt, daß der pH-Wert auf 6,5 ansteigt (etwa 29 ml). Dann wird der pH-Wert mit 2n Natronlauge auf 9,2 eingestellt, und 50 ml 10 %ige Natriumhydrogencarbonatlösung werden zugegossen. Nach 3 Stunden wird filtriert und getrocknet. 39,78 g Produkt werden erhalten, welches imsgesamt 14,96 g OTC enthält. Das entspricht einer Ausbeute von 76 %.

**Beispiel 4**

Zu 1704 g OTC-Fermentbrühe (enthaltend insgesamt 16,040 g OTC) werden bei Raumtemperatur unter Rühren 153 ml 10 %ige Oxalsäure gegeben (= 90 ml/kg). Dabei sinkt der pH-Wert auf 2. Das Gemisch wird filtriert und das Mycel mit 426 ml Wasser (= 250 ml/kg) gewaschen. Filtrat umd Waschwasser werden vereinigt. Die erhaltenen 1970 ml Flüssigkeit enthalten 13,794 g OTC. Der pH-Wert der Lösung wird mit 2n Natronlauge auf 4,5 eingestellt. Dann werden 52 ml 10 %ige CaCl$_2$-Lösung und anschließend bis pH 6,5 im Verhältnis 1:3 mit Wasser verdünntes Wasserglas zugegeben (etwa 21 ml). Nach einstündigem Rühren wird der pH-Wert mit 2n Natronlauge auf 9 eingestellt. Man rührt noch drei Stunden, filtriert das Produkt ab und trocknet es. 28,29 g Produkt werden erhalten, das 13,040 g (46,1 %) OTC enthält, was einer Ausbeute von 81 % entspricht.

**Beispiel 5**

Zu 2124 g OTC-Fermentbrühe (enthaltend insgesamt 18,035 g OTC) werden bei Raumtemperatur 127 ml eines Schwefelsäure-Oxalsäure-Gemisches gegeben, das 11 Gew.-% Schwefelsäure und 1,5 Gew.-% Oxalsäure enthält. Der pH-Wert sinkt auf 2. Das Gemisch wird eine Stunde lang gerührt, dann wird Perlit in einer Menge von 5 g/kg zugegeben und das Gemisch filtriert. Das Mycel wird mit 530 ml Wasser gewaschen. Die durch Vereinigung von Filtrat und Waschwasser erhaltenen 2390 ml Lösung enthalten 16,646 g OTC. Der pH-Wert der Lösung wird mit 2n Natronlauge auf 4,5 eingestellt. Zu der Lösung werden 52,4 ml im Verhältnis 1:3 mit Wasser verdünntes Wasserglas und 160 ml 10 %ige CaCl$_2$-Lösung gegeben. Der danach etwa 6,5 betragende pH-Wert wird mit 2n Natronlauge auf 9,2 eingestellt. Zu der Lösung werden 51 ml 10 %ige Natriumhydrogencarbonatlösung gegeben. Nach 2 Stunden wird filtriert und getrocknet. 43,69 g des insgesamt 16,033 g OTC enthaltenden Komplexsalzes werden erhalten. Ausbeute an OTC: 89 %.

**Beispiel 6**

Aus 2109 g OTC-Fermentbrühe (enthaltend insgesamt 20,035 g OTC) werden auf die im Beispiel 5 beschriebene Weise 2373 ml Filtrat (enthaltend 18,432 g OTC) hergestellt. Der pH-Wert des Filtrats wird mit 2n Natronlauge auf 4,5 eingestellt (etws 88 ml), dann wird nach Zugabe von 180 ml 10 %iger CaCl$_2$-Lösung der pH-Wert zunächst mit im Verhältnis 1:3 verdünntem Wasserglas auf 6,5 (etwa 35 ml sind erforderlich), dann mit 2n Natronlauge auf 9,2 (etwa 79 ml) eingestellt. Dann wird ein dem zugesetzten Wasserglasvolumen entsprechendes Volumen an 10 %iger Natriumhydrogencarbonatlösung (etwa 35 ml) zugesetzt. Nach 2stündigem Rührem wird filtriert. Das Produkt wird mit Heiß, luft (100-110°C) getrocknet. 43,6 g Komplexsalz werden erhalten, welches insgesamt 17,43 g OTC enthält. Das entspricht einer Ausbeute von 87 %.

**Beispiel 7**

Zu 2065 g OTC-Fermentbrühe (enthaltend insgesamt 21,578 g OTC) werden unter Rühren 0,63 g Sterogenol (Cetylpyridiniumbromid) in Form einer mit warmem Wasser bereiteten 10 %igen Lösung zugegeben. Der pH-Wert des Gemisches wird mit dem auch in Beispiel 5 verwendeten Säuregemisch auf 5,3-5,6 eingestellt. Das Gemisch wird innerhalb von 30 Minuten auf 40 °C erwärmt und bei dieser Temperatur 2 Stunden lang gerührt. Nach dem Abkühlen auf Raumtemperatur kann die vorbehandelte Fermentbrühe nach einem beliebigen der Beispiele 3-6 weiterverarbeitet werden. Je nach gewünschtem Reinheitsgrad werden Ausbeuten von 80-90 % erzielt.

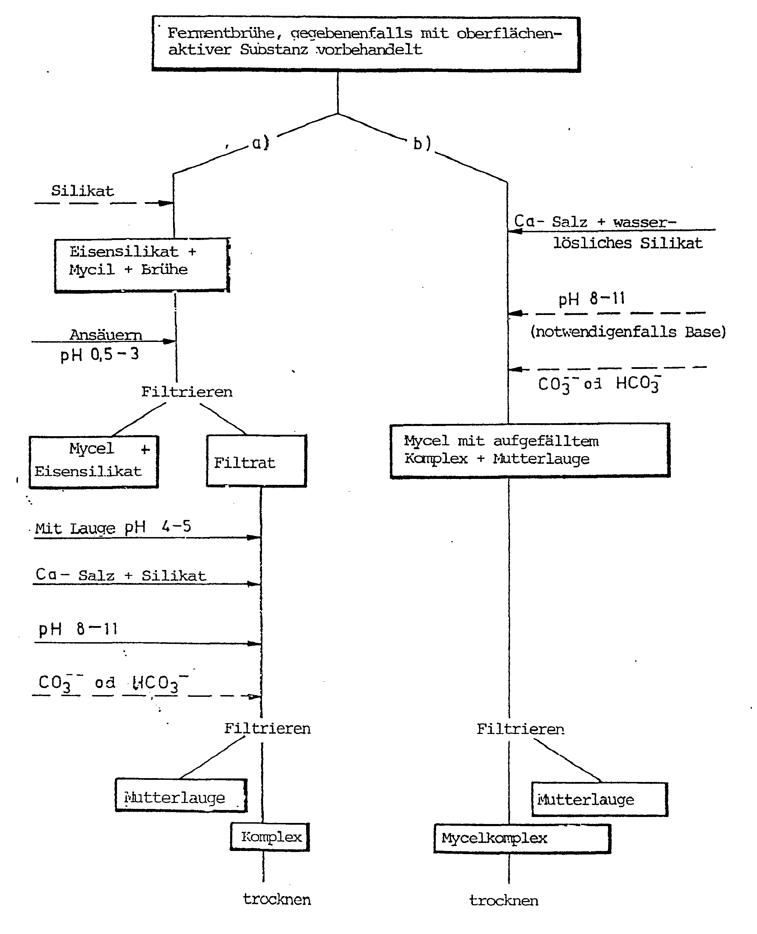

**Patentansprüche**

1. Verfahren zur Herstellung eines Oxytetracyclim-Komplexsalzes aus der

Fermentbrühe, dadurch <u>gekennzeichnet</u>, daß man die gegebenenfalls mit einer oberflächenaktiven Substanz vorbehandelte Fermentbrühe

a) nach einer gegebenenfalls durchgeführten Vorbehandlung mit einem wasserlöslichen Silikat auf pH $^{0,5}$-3,$^0$ ansäuert, danach - gegebenenfalls unter Verwendung einer Filtrierhilfe - vom Mycel abfiltriert, den pH-Wert des Filtrates mit Lauge auf 4-5 einstellt und nach Zugabe eines Calciumsalzes oder in Gegenwart von Calciumionen ein wasserlösliches Silikat zusetzt, gegebenenfalls ein wasserlösliches Carbonat oder Hydrogencarbonat zugibt und nach längerem Rühren das gebildete Oxytetracyclin-Calciumsilikat-Komplexsalz gegebenenfalls in Gegenwart eines Filtrierhilfsstoffes abfiltriert, oder

b) mit einem Calciumsalz und einem wasserlöslichen Silikat vermischt, erforderlichenfalls den pH-Wert mit Lauge auf 8-11 einstellt, gegebenenfalls dem Gemisch ein wasserlösliches Carbonat oder Hydrogencarbonat zusetzt und nach längerem Rühren das auf das Mycel aufgefällte Oxytetracyclin-Calciumsilikat-Komplexsalz gegebenenfalls in Gegenwart eines Filtrierhilfsstoffes abfiltriert,

und das nach a) oder b) erhaltene Oxytetracyclin-Calciumsilikat-Komplexsalz bei 20-120 °C trocknet.

2. Verfahren nach Anspruch 1a), dadurch gekennzeichnet, daß man zum Ansäuern auf pH 0,5-3,0 ein Gemisch aus Oxalsäure und Schwefelsäure verwendet.

3. Verfahren nach Anspruch 1a), dadurch gekennzeichnet, daß man als wasserlösliches Silikat im Verhältnis von etwa 1:3 mit Wasser verdünntes handelsübliches Natronwasserglas in einer Menge von auf 1 kg Fermentbrühe bezogen 1-20 ml, vorzugsweise 4-9 ml, verwendet.

4. Verfahren nach Anspruch 1b), dadurch gekennzeichnet, daß man als wasserlösliches Silikat im Verhältnig mit Wasser verdünntes Natronwasserglas in einer Menge von auf 1 kg Fermentbrühe bezogen 5-100 ml, vorzugsweise 8-12 ml, einsetzt.

5. Verfahren nach Anspruch 1, dadurch gekennzeichnet, daß man als Calciumsalz Calciumchlorid verwendet.

6. Verfahren nach Anspruch 1, dadurch gekennzeichnet, daß man als Hydrogencarbonat Natriumhydrogencarbonat verwendet.

## Claims

1. A process for the manufacture of an oxytetracycline complex salt from a fermentation broth, characterised in that the fermentation broth, which is optionally pretreated with a surfactant,

a) is acidified to a pH value of 0.5-3.0 after an optionally performed pretreatment with a water-soluble silicate, then - in given cases by utilising a filtration aid - mycelium is filtered out from it, the pH value of the filtrate is adjusted to 4-5 by means of an alkali and after addition of a calcium salt or in the presence of calcium ions a water-soluble silicate is added, optionally a water-soluble carbonate or bicarbonate is added, and after lengthy agitation the formed oxytetracyclinecalcium silicate-complex salt is filtered, in given cases in the presence of a filtration aid material, or

b) is mixed with a calcium salt and a water-soluble silicate; if required, the pH value is adjusted to 8-11 with an alkali, optionally a water-soluble carbonate or bicarbonate is added to the mixture, and after lengthy agitation the oxytetracycline-calcium silicate-complex salt deposited on the mycelium is filtered off, optionally in the presence of a filtration aid material,

and the oxytetracycline-calcium silicate-complex salt obtained according to a) or b) is dried at 20-120° C.

2. A process according to claim 1a), characterised in that a mixture of oxalic acid and sulphuric acid is used for the acidification to a pH value of 0.5-3.0.

3. A process according to claim 1a), characterised in that as a water-soluble silicate commercially available sodium water glass diluted with water in the ratio of about 1:3 is used in an amount of 1-20 ml, preferably 4-9 ml, based on 1 kg fermentation broth.

4. A process according to claim 1b), characterised in that as a water-soluble silicate water-diluted sodium water glass is used in an amount of 5-100 ml, preferably 8-12 ml, based on 1 kg fermentation broth.

5. A process according to claim 1, characterised in that calcium chloride is used as the calcium salt.

6. A process according to claim 1, characterised in that sodium bicarbonate is used as the bicarbonate.

## Revendication

1 - Procédé de préparation d'un sel complexe d'oxytétracycline à partir du bouillon de fermentation, caractérisé en ce que, partant du bouillon de fermentation éventuellement traité au préalable par une substance tensioactive,

a) après un traitement préalable éventuel par un silicate soluble dans l'eau, on acidifie à pH 0,5-3,0 puis -éventuellement en utilisant un produit auxiliaire de filtration - on sépare le mycélium par filtration, on règle le pH du filtrat à un niveau de 4-5 à l'aide d'une lessive alcaline et, après addition d'un sel de calcium ou en présence d'ions calcium, on ajoute un silicate soluble dans l'eau, on ajoute encore éventuellement un carbonate ou bicarbonate soluble dans l'eau et, après agitation prolongée, on sépare par filtration le sel complexe d'oxytétracycline-silicate de calcium formé, éventuellement en présence d'un produit auxiliaire de filtration, ou bien

b) on mélange avec un sel de calcium et un silicate soluble dans l'eau, on règle à pH 8-11 à l'aide d'une lessive alcaline si c'est nécessaire, on ajoute éventuellement au mélange un carbonate ou bicarbonate soluble dans l'eau et, après agitation prolongée, on sépare par filtration le sel complexe d'oxytétracyclinesilicate de calcium qui a précipité sur le mycélium, éventuellement en présence d'un produit auxiliaire de filtration, et on sèche à une température de 20 à 120° C le sel complexe d'oxytétracycline-silicate de calcium obtenu en a) ou b).

2 - Procédé selon la revendication 1a), caractérisé en ce que, pour acidifier à un pH de 0,5 à 3,0, on utilise un mélange d'acide oxalique et d'acide sulfurique.

3 - Procédé selon la revendication 1a), caractérisé en ce que l'on utilise en tant que silicate soluble dans l'eau de la lessive de silicate de sodium du commerce, diluée par l'eau à un rapport d'environ 1:3, en quantité de 1 à 20 ml, de préférence de 4 à 9 ml pour 1 kg du bouillon de fermentation.

4 - Procédé selon la revendication 1b), caractérisé en ce que l'on utilise en tant que silicate soluble dans l'eau de la lessive de silicate de sodium diluée par l'eau au rapport de 1:1 en quantité de 5 à 100 ml, de préférence de 8 à 12 ml pour 1 kg du bouillon de fermentation.

5 - Procédé selon la revendication 1, caractérisé en ce que le sel de calcium utilisé est le chlorure de calcium.

6 - Procédé selon la revendication 1, caractérisé en ce que le bicarbonate utilisé est le bicarbonate de sodium.

.

Fermentbrühe, gegebenenfalls mit oberflächenaktiver Substanz vorbehandelt

a)      b)

Silikat

Ca- Salz + wasserlösliches Silikat

Eisensilikat +
Mycil + Brühe

pH 8—11

(notwendigenfalls Base)

Ansäuern
pH 0,5 - 3

$CO_3^-$ od $HCO_3^-$

Filtrieren

Mycel +
Eisensilikat

Filtrat

Mycel mit aufgefälltem
Komplex + Mutterlauge

Mit Lauge pH 4-5

Ca- Salz + Silikat

pH 8—11

$CO_3^-$ od $HCO_3^-$

Filtrieren

Filtrieren

Mutterlauge

Mutterlauge

Komplex

Mycelkomplex

trocknen

trocknen

1